# EUROPEAN PATENT APPLICATION

(11) **EP 4 014 852 A1**
(43) Date of publication of application: **22.06.2022**
(21) Application number: 19945505.6
(22) Date of filing: 13.11.2019
(51) Int. Cl.: A61B 5/0205, A61B 5/03, A61B 5/00, A61B 5/1455

(54) **DETECTION PROBE AND FETAL MONITOR**

(30) Priority: 16.09.2019 CN 201910870888
(71) Applicant: Edan Instruments, Inc., Shenzhen, Guangdong 518122 (CN)
(72) Inventor: LUO, Chong, Shenzhen, Guangdong 518122 (CN); CHEN, Dewei, Shenzhen, Guangdong 518122 (CN); DONG, Lichao, Shenzhen, Guangdong 518122 (CN)
(74) Representative: Zacco Norway AS
(86) International application number: PCT/CN2019/117754
(87) International publication number: WO 2021/051571

(57) **Abstract**

A detection probe and a fetal monitor. The detection probe comprises a probe body (2) and a mounting structure (1), detachably mounted on the probe body (2) and having a heart rate sensor for heart rate detection, a socket (4) for data transmission, and a lead line (5) for connecting the heart rate sensor and the socket (4) arranged thereon. A variety of functions can be achieved by means of a single detection device and thus the degree of integration is high; moreover, when the heart rate sensor is failed or aged, because the mounting structure (1) can be separated from the probe body (2), it is only necessary to repair and replace a corresponding part, so that the maintenance is convenient and the cost is reduced. Furthermore, the mounting structure (1) can also product the probe body (2) to some extent, and thus the anti-dropping capability of the detection probe is improved.

## Description

### TECHNICAL FIELD

The present application relates to the technical field of medical devices, in particular to a detection probe and a fetal monitor.

### BACKGROUD

The principle for acquiring a fetal heart rate in a fetal monitor is mainly based on an ultrasonic Doppler effect, ultrasonic waves may be emitted after meeting density-changed tissues, frequency offsets of the ultrasonic waves may occur when a moving object is reflected, these frequency offsets may be demodulated, these information represents the motion conditions of the tissues, a heart-relevant motion frequency may be obtained by calculating a motion frequency, and thus, a heart rate may be calculated. At present, all echoes within an irradiation range of a probe may be demodulated and calculated in the prior art, and therefore, it is very possible to measure abdominal blood vessels of a mother; and when the heart rate of the mother is relatively high, it is very easy to recognize the heart rate of the mother as the fetal heart rate by mistake to result in misdiagnosis.

In order to reduce the risk that such a problem occurs, the fetal monitor is always adopted to synchronously measure the heart rate of the mother while measuring the fetal heart rate and compare the fetal heart rate with the heart rate of the mother so as to judge whether a source of the fetal heart rate is correct.

Commonly, the heart rate of the mother is measured by adopting a blood oxygen or electrocardio method, and both the blood oxygen method and the electrocardio method need to adopt a sensor. The blood oxygen method generally adopts a manner of a finger clip, an ear clip or a forehead patch, the electrocardio method generally adopts a manner that a lead line is pasted to an electrode sheet, cables may be used in the both manners, and when these sensors and the cables are used, a pregnant woman may not feel very comfortable, and activities of the pregnant woman may be disturbed.

For this purpose, a blood oxygen sensor is integrated into a uterine contraction probe in some solutions of blood oxygen technologies. When the uterine contraction probe is used, the sensor may cling to the skin of the pregnant woman, blood oxygen signals of abdominal capillary blood vessels of the pregnant woman are measured by using a blood oxygen technology, and thus, the heart rate of the pregnant woman is calculated. In such a manner, the cable problem in a traditional solution is solved, however, the sensor is integrated in the probe, the blood oxygen sensor adopts an LED, such an element may generate luminous decay and is relatively short in service life and easy to damage, maintenance is not facilitated due to integration inside the probe, and the overall probe is required to be replaced, which brings additional maintenance cost for a user.

### SUMMARY

Therefore, the technical problem to be solved by the present application is to overcome defects in the prior art that a heart rate sensor integrated in other probe bodies is easily damaged, maintenance is not facilitated, and the overall probe is require to be replaced, which brings additional maintenance cost for a user. Therefore, a detection probe and a fetal monitor are provided.

A detection probe provided by the present application includes a probe body and a mounting structure, detachably mounted on the probe body; and having a heart rate sensor for heart rate detection, a socket for data transmission, and a lead line for connecting the heart rate sensor and the socket arranged thereon.

Optionally, the mounting structure is a housing structure matched with a shell of the probe body and is detachably buckled on the probe body.

Optionally, the housing structure includes an acting surface fitted with the probe body, which is fitted to a user during detection, and has the heart rate sensor arranged thereon; and a connecting part connected with the acting surface, which wraps a peripheral edge of the probe body.

Optionally, the heart rate sensor is an electrocardio electrode arranged on the acting surface.

Optionally, the electrocardio electrode includes a main left arm electrode and a main right arm electrode respectively configured to acquire electrocardio signals of a left arm and a right arm.

Optionally, the detection probe further includes at least one auxiliary left arm electrode configured to acquire an electrocardio signal in a left arm area and spaced for a set distance from the main left arm electrode and at least one auxiliary right arm electrode configured to acquire an electrocardio signal in a right arm area and spaced for a set distance from the main right arm electrode.

Optionally, two auxiliary left arm electrodes which are respectively a first auxiliary left arm electrode and a second auxiliary left arm electrode and two auxiliary right arm electrodes which are respectively a first auxiliary right arm electrode and a second auxiliary right arm electrode are respectively arranged.

Optionally, a lead axis formed from the main left arm electrode and the second auxiliary right arm electrode is perpendicular to a lead axis formed from the main right arm electrode and the second auxiliary left arm electrode.

Optionally, the detection probe further includes a right leg driving electrode configured to improve the common-mode rejection ability of a system.

Optionally, the lead line is arranged between the acting surface and the probe body, and the housing structure is provided with a through hole allowing the heart rate sensor to pass through.

Optionally, the lead line is a flexible printed circuit (FPC) line or is of a conductive structure which is printed on the side, facing the probe body, of the housing structure.

Optionally, the heart rate sensor is a blood oxygen photoplethysmographic sensor.

Optionally, the probe body is an outer uterine contraction pressure probe, a fetal heart probe or an inner monitoring probe.

The present application further provides a fetal monitor including the detection probe according to any one of the above-mentioned descriptions.

The technical solutions of the present application have the following advantages.
1. The detection probe provided by the present application includes the probe body and the mounting structure, detachably mounted on the probe body; and having the heart rate sensor for heart rate detection, the socket for data transmission, and the lead line for connecting the heart rate sensor and the socket arranged thereon. According to such a design, a variety of functions can be achieved by means of the single detection probe, and thus, the degree of integration is high; moreover, when the heart rate sensor is failed or aged, because the mounting structure can be separated from the probe body, it is only necessary to repair and replace a corresponding part, so that the maintenance is convenient and the cost is reduced. Furthermore, the mounting structure can also protect the probe body to some extent, and thus, the anti-dropping capability of the detection probe is improved.
2. According to the detection probe provided by the present application, the mounting structure is a housing structure matched with the shell of the probe body and is detachably buckled on the probe body. Due to such a design, the probe body is detachably mounted, meanwhile, the probe body is also protected, and thus,the anti-dropping capability of the probe body is improved.
3. According to the detection probe provided by the present application, the electrocardio electrode includes seven electrocardio electrodes arranged around the center of the acting surface, by combining the seven electrocardio electrodes in pairs, signals forming different angles with an electrocardio axis may be acquired to cover the range of a standard electrocardio axis, thereby reducing operation actions of the user; and an optimal signal channel may be automatically adjusted to an optimal electrocardio conducting direction with specific to the position of the probe by virtue of software and an algorithm, so that it is ensured that maximum and optimal signals are acquired, the stability and reliability of the overall monitoring process are guaranteed, and influences brought by artificial operation are reduced.

### BRIEF DESCRIPTION OF THE DRAWINGS

In order to more clearly describe the technical solutions in the specific implementations of the present application or the prior art, the accompanying drawings required to be used in the descriptions of the specific implementations or the prior art will be briefly introduced below. Apparently, the accompanying drawings in the following descriptions are some implementations of the present application, those of ordinary skill in the art may obtain other accompanying drawings from these accompanying drawings without creative work.
Fig. 1 is a schematic diagram showing a structure of a detection probe provided in a first implementation of the present application;
Fig. 2 is a schematic diagram showing structures of a mounting structure and an electrocardio electrode as shown in Fig. 1;
Fig. 3 is a schematic diagram showing structures of the mounting structure, a lead line and a socket as shown in Fig. 1;
Fig. 4 is a diagram showing a working circuit of the electrocardio electrode in Fig. 2;
Fig. 5 is a schematic diagram showing a common lead direction for a frontal plane of a human body and a horizontal plane;
Fig. 6 is a schematic diagram showing structures of a mounting structure and an electrocardio electrode in a detection probe provided in a second implementation of the present application; and
Fig. 7 is a process diagram showing an embodiment of a heart rate acquiring method provided by the present application.

Description for reference numerals in the accompanying drawings:
1-mounting structure, 2-probe body, 3-acting surface, 4-socket, and 5-lead line.
101-first differential amplifier, 102-second differential amplifier, 103-third differential amplifier, 104-fourth differential amplifier, 105-fifth differential amplifier, 106-sixth differential amplifier, and 107-processing unit.
LA; main left arm electrode, LA1: first auxiliary left arm electrode, LA2: second auxiliary left arm electrode;
RA: main right arm electrode, RA1: first auxiliary right arm electrode, LA2: second auxiliary right arm electrode; and
RL: right leg driving electrode.

### DETAILED DESCRIPTION

The technical solutions of the present application will be described clearly and completely below with reference to the accompanying drawings. Obviously, the described embodiments are only a part of embodiments instead of all embodiments of the present application. All other embodiments obtained by those of ordinary skill in the art based on the embodiments of the present application without creative work shall fall within the protection scope of the present application.

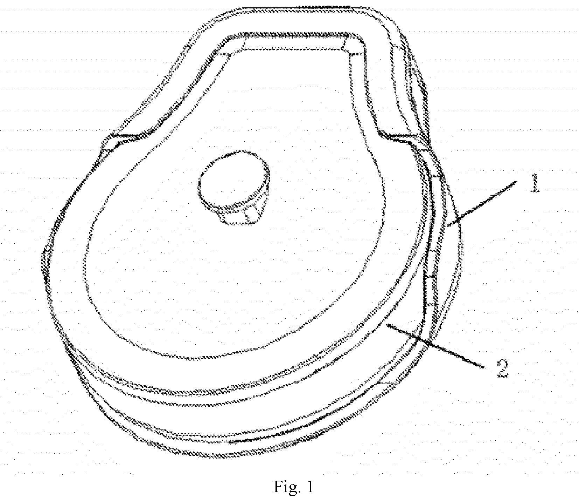

Fig. 1 to Fig. 4 show an embodiment of a detection probe provided by the present application.

The detection probe includes a probe body 2, a mounting structure 1 and a heart rate sensor. In the present embodiment, the probe body 2 adopts an outer uterine contraction pressure probe for detecting a uterine contraction pressure. However, the probe body 2 is not limited to the outer uterine contraction pressure probe, may be a probe, such as a fetal heart probe or an inner monitoring probe, for measuring other parameters of an adult or a fetus, and is not limited to a wired probe or a wireless probe.

The probe body 2 includes an inner side surface close to a user, an outer side surface opposite to the inner side surface and a peripheral surface connected with the inner side surface and the outer side surface. The inner side surface includes a detection area fitted to the user to achieve a detection function of the probe body 2, and the outer side surface is provided with a bandage buckle by which the detection probe is banded on the abdomen by the user by using a bandage.

The mounting structure 1 is a housing structure matched with a shell of the probe body 2. The housing structure may directly sleeve the exterior of the probe body 2 and be combined with the probe body 2 into a whole and may be freely detached from the probe body 2 so as to be separated from the probe body 2. Specifically, the housing structure includes an acting surface 3 fitted with the inner side surface of the probe body 2 and a connecting part connected with the edge of the acting surface 3. The acting surface 3 is arranged to be hollow corresponding to the detection area on the inner side surface so that the probe body 2 achieves the detection function. The connecting part wraps the peripheral surface of the probe body 2, a tail end is provided with a buckling part extending to the central position of the probe body 2 for a set distance, and the buckling part is used for buckling connection between the housing structure and the probe body 2. When being mounted on the probe body 2, the housing structure naturally becomes a protective housing of the probe body 2 to further protect the probe body 2, and thus, the anti-dropping capability of the detection probe is improved.

In order to facilitate dismounting and mounting between the housing structure and the probe body 2, the housing structure is made of a deformable elastic material such as a plastic or silicon rubber material, and obviously, the material of the housing is not limited to the above-mentioned two materials. The thickness of the housing structure is best not to excessively increase the overall size of the detection probe or affect the use experience and may be determined according to the size of the probe body 2 and the design size of the detection probe, and in the present embodiment, the thickness is 1 mm.

The acting surface 3 is fitted to the user during detection, and the heart rate sensor is arranged on the acting surface 3. The acting surface 3 is provided with a through hole allowing the heart rate sensor to pass through and a hollow area corresponding to the detection area of the probe body 2. In the present embodiment, the hollow area is of a round structure, and the through hole is formed in the periphery of the hollow area.

The heart rate sensor is an electrocardio electrode arranged on the acting surface 3. The electrocardio electrode is made of a conducctive material which is not limited to copper, gold, stainless steel and carbon; and meanwhile, the electrocardio electrode may be designed to be square, planar, dotted and the like, and the shape of the through hole is designed to correspond to the shape of the electrocardio electrode.

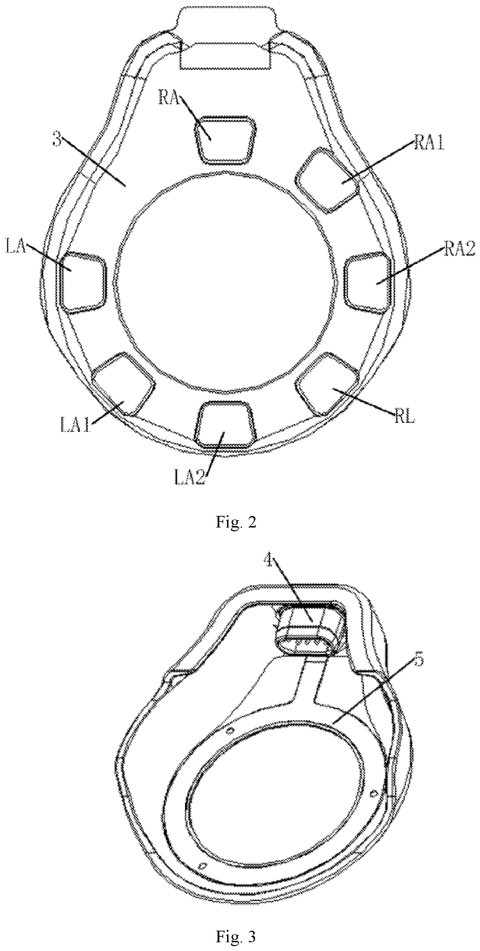

As shown in Fig.2, in the present embodiment, the electrocardio electrode includes seven electrocardio electrodes arranged around the center of the acting surface 3 to form an included angle for the heart of an adult. The seven electrocardio electrodes are respectively a main left arm electrode LA, a first auxiliary left arm electrode LA1, a second auxiliary left arm electrode LA2, a right leg driving electrode RL, a second auxiliary right arm electrode RA2, a first auxiliary right arm electrode RA1 and a main right arm electrode RA. Due to a difference of human bodies, a distance and an angle among the electrocardio electrodes may be freely set without specific limitations.

Wherein the main left arm electrode LA and the main right arm electrode RA are fixed electrodes and are respectively configured to acquire electrocardio signals of a left arm and a right arm of the adult.

The first auxiliary left arm electrode LA1 and the second auxiliary left arm electrode LA2 are arranged to be spaced from the main left arm electrode LA for a certain distance and are regional electrodes for acquiring an electrocardio signal in a left arm area of the adult. The first auxiliary left arm electrode LA1 differs from the main right arm electrode RA, and the second auxiliary left arm electrode LA2 differs from the main right arm electrode RA, so that signals different from differential signals of the main left arm electrode LA and the main right arm electrode RA may be acquired, and signals forming different angles with an electrocardio axis may be acquired.

The first auxiliary right arm electrode RA1 and the second auxiliary right arm electrode RA2 are arranged to be spaced from the main right arm electrode RA for a certain distance and are regional electrodes for acquiring an electrocardio signal in a right arm area of the adult. The main left arm electrode LA differs from the first auxiliary right arm electrode RA1, and the main left arm electrode LA differs from the second auxiliary right arm electrode RA2, so that signals different from differential signals of the main left arm electrode LA and the main right arm electrode RA may be acquired, and signals forming different angles with the electrocardio axis may be acquired.

Each cardiac cycle of the heart of a human body is accompanied by depolarization and repolarization processes of myocardial cells, and such bioelectric variation is reflected to the body surface through conductive tissues and body fluid surrounding the heart, so that each part of the body generates regular electric variation in each cardiac cycle, electrodes are placed on appropriate positions of limbs or the body to detect electric signals on the different parts, recordable electric activities of the heart are amplified and traced, and thus, an electrocardiograph (ECG) is obtained.

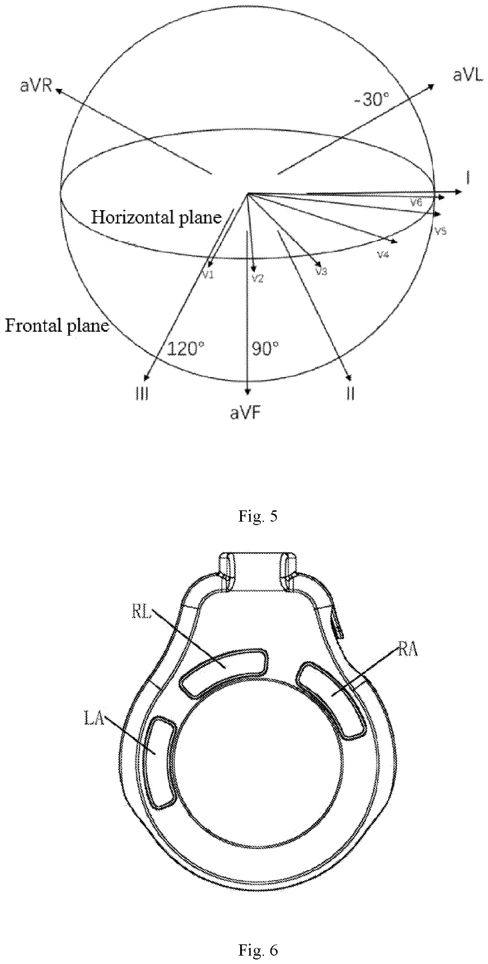

The waveform size of the ECG picked up by the electrocardio electrode is decided by the length of a projection of the electrocardio axis (a mean QRS vector) on a lead axis. Reference is made to Fig. 5, normally, if the electrocardio axis of the adult is located on a frontal plane in a three-dimensional space, the electrocardio axis is leftward and downward, that is, the electrocardio axis is located between a lead I and a lead aVF; and if the electrocardio axis of the adult is located on a horizontal plane, the electrocardio axis is backward, that is, the electrocardio axis is located between V6 and negative V2. The distribution range of an electrocardio axis of a frontal plane of a normal person is 0 DEG to 90 DEG according to domestic standards.

Reference is made to Fig. 2, a lead axis formed from the main left arm electrode LA and the second auxiliary right arm electrode RA2 is perpendicular to a lead axis formed from the main right arm electrode RA and the second auxiliary left arm electrode LA2, so that detection angles of a combination of the main left arm electrode LA and the second auxiliary right arm electrode RA2 and a combination of the second auxiliary left arm electrode LA2 and the main right arm electrode RA are 90 DEG, and then, the standard range of the electrocardio axis may be covered.

The right leg driving electrode RL is configured to improve the common-mode rejection of a system. A common-mode voltage on the surface of a human body is acquired by the right leg driving electrode RL and is then returned to the human body in a negative feedback amplification manner, so that the effect of neutralizing common-mode interference is achieved, and the common-mode voltage is rejected fundamentally.

The housing structure is provided with a socket 4 and a lead line 5. Reference is made to Fig. 3, the socket 4 is configured to transmit physiological data and and realizes signal connection in a manner of metal pin contact or other manners. The lead line 5 adopts a flexible printed circuit (FPC) line and is configured to connect the electrocardio electrode and the socket 4 so as to transmit relevant physiological signals. The lead line 5 is connected with the electrocardio electrode in a manner of welding or conductive adhesive. The lead line 5 is placed between the probe body 2 and the housing structure, and the housing structure is utilized to protect the lead line 5. The lead line 5 may also be made by using a process such as cable and silver paste printing.

Known from the above-mentioned description, according to the detection probe provided by the present application, a variety of functions can be achieved by means of a single detection probe, so that the degree of integration is high, and the use experience of the user is improved. Moreover, when the heart rate sensor is failed or aged, because the mounting structure can be separated from the probe body, it is only necessary to repair and replace a corresponding part, so that the defects in the prior art that maintenance is not facilitated, and the overall detection probe is required to be replaced, which brings additional maintenance cost for the user are overcome, the maintenance is convenient, and the cost is reduced.

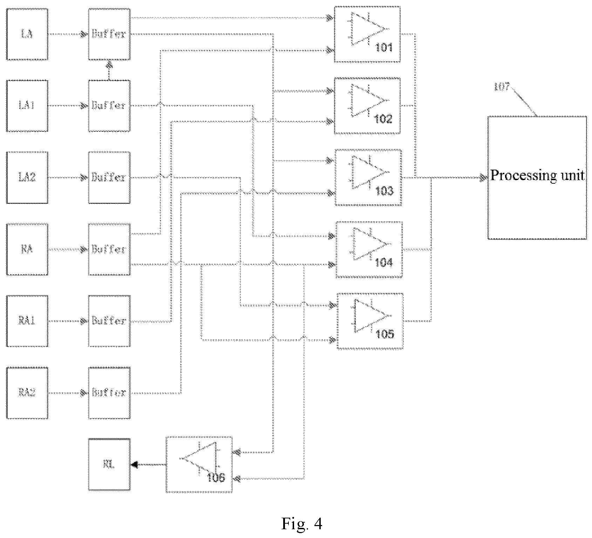

Fig. 4 shows a diagram showing a working circuit of the electrocardio electrode in the detection probe provided by the present application. Wherein the main left arm electrode LA, the first auxiliary left arm electrode LA1, the second auxiliary left arm electrode LA2, the main right arm electrode RA, the first auxiliary right arm electrode RA1 and the second auxiliary right arm electrode RA2 are respectively connected with corresponding buffer amplifiers.

Due to different skin contact resistances, weak electrocardio signals and the like, if the electrocardio signals of the electrocardio electrodes are directly amplified, the amplifiers are required to have extremely high input impedance, which is very difficult to achieve in hardware in the prior art. By setting the buffer amplifiers, impedance matching between the signals and the input ends of differential amplifiers is realized, so that it is ensured that the electrocardio signals are not distorted or interfered.

The main left arm electrode LA and the main right arm electrode RA are respectively connected to two input ends of a first differential amplifier 101 by corresponding buffer amplifiers, and an output end of the first differential amplifier 101 is connected to a processing unit 107, so that a differential circuit of the main left arm electrode LA and the main right arm electrode RA is formed, and difference, amplification and signal sampling of the electrocardio signals are achieved.

The main left arm electrode LA and the first auxiliary right arm electrode RA1 are respectively connected to two input ends of a second differential amplifier 102 by corresponding buffer amplifiers, and an output end of the second differential amplifier 102 is connected to the processing unit 107, so that a differential circuit of the main left arm electrode LA and the first auxiliary right arm electrode RA1 is formed, and difference, amplification and signal sampling of the electrocardio signals are achieved.

The main left arm electrode LA and the second auxiliary right arm electrode RA2 are respectively connected to two input ends of a third differential amplifier 103 by corresponding buffer amplifiers, and an output end of the third differential amplifier 103 is connected to the processing unit 107, so that a differential circuit of the main left arm electrode LA and the second auxiliary right arm electrode RA2 is formed, and difference, amplification and signal sampling of the electrocardio signals are achieved.

The first auxiliary left arm electrode LA1 and the main right arm electrode RA are respectively connected to two input ends of a fourth differential amplifier 104 by corresponding buffer amplifiers, and an output end of the fourth differential amplifier 104 is connected to the processing unit 107, so that a differential circuit of the first auxiliary left arm electrode LA1 and the main right arm electrode RA is formed, and difference, amplification and signal sampling of the electrocardio signals are achieved.

The second auxiliary left arm electrode LA2 and the main right arm electrode RA are respectively connected to two input ends of a fifth differential amplifier 105 by corresponding buffer amplifiers, and an output end of the fifth differential amplifier 105 is connected to the processing unit 107, so that a differential circuit of the second auxiliary left arm electrode LA2 and the main right arm electrode RA is formed, and difference, amplification and signal sampling of the electrocardio signals are achieved.

The main left arm electrode LA and the main right arm electrode RA are respectively connected to two input ends of a sixth differential amplifier 106 by corresponding buffer amplifiers, and an output end of the sixth differential amplifier 106 is connected to the right leg driving electrode RL, so that a right leg driving circuit is formed. A common-mode voltage on the surface of a human body is acquired by the right leg driving circuit by virtue of the right leg driving electrode RL and is then returned to the human body in a negative feedback amplification manner, so that the effect of neutralizing common-mode interference is achieved, and the common-mode voltage is rejected fundamentally.

The differential circuit includes two input ends and an output end, a voltage difference of the two input ends is amplified with a fixed gain, and thus, the output power of a signal is increased. An energy source is acquired from a power supply, so that the waveform of an output signal is controlled to be consistent with that of an input signal, but the output signal has a greater amplitude.

The processing unit is configured to judge the quality of a signal acquired by each channel in fact so as to decide to adopt data of the channel with the optimal signal quality to perform relevant calculations.

By adopting the above-mentioned circuit, difference, amplification and sampling of electrocardio signals of different electrode combinations may be achieved. By combining the electrodes in pairs, signals forming different angles with the electrocardio axis may be acquired to cover the range of a standard electrocardio axis, thereby reducing operation actions of the user; and the quality of the signal is judged to decide to adopt the electrode combination with the optimal signal quality to perform relevant calculations. Therefore, an optimal electrocardio conducting direction is automatically adjusted, so that it is ensured that maximum and optimal signals are acquired, the stability and reliability of the overall monitoring process are guaranteed, and influences brought by artificial operation are reduced.

As another implementation, the heart rate sensor may be replaced with a blood oxygen photoplethysmographic sensor. The heart rate of the user is calculated by detecting reflected light caused by abdominal blood vessels of the user. The heart rate sensor in the present application may also be various types of heart rate sensors which are used at the same time, and the types of the heart rate sensors are not limited to electrocardio and blood oxygen.

As further implementation, as shown in Fig. 6, the electrocardio electrode includes three electrocardio electrodes arranged around the center of the acting surface 3. The three electrocardio electrodes are respectively a main left arm electrode LA, a right leg driving electrode RL, a main right arm electrode RA. The main left arm electrode LA and the main right arm electrode RA are fixed electrode and are configured to respectively acquire electrocardio signals of a left arm and a right arm of an adult.

It can be seen herefrom that the detection probe provided by the present application may only retain the fixed electrodes, and the heart rate of a user may be acquired by acquiring the electrocardio signals of the fixed electrodes. When the detection probe is used, a probe electrode system may be slightly adjusted according to amplitudes of the electrocardio signals to ensure that an included angle of the lead axis and the electrocardio axis in a three-dimensional space is relatively small, so that forward electrocardio signals which are relatively large are acquired. Apparently, in order to reduce the operation actions of the user, avoid influences of the operation of the user on a monitoring structure and acquire better signals, a multi-electrode design solution in the above-mentioned implementation is adopted as a preferred solution.

As yet further implementation, the right leg driving electrode RL is configured to improve the common-mode rejection ability of a system and may be omitted in some implementations. For example, the electrocardio electrode includes six electrocardio electrodes arranged around the center of the acting surface 3 to form an included angle for the heart of an adult. The six electrocardio electrodes are respectively a main left arm electrode LA, a first auxiliary left arm electrode LA1, a second auxiliary left arm electrode LA2, a second auxiliary right arm electrode RA2, a first auxiliary right arm electrode RA1 and a main right arm electrode RA.

As further another implementation, the electrocardio electrode includes five electrocardio electrodes arranged around the center of the acting surface 3 to form an included angle for the heart of an adult. The five electrocardio electrodes are respectively a main left arm electrode LA, a first auxiliary left arm electrode LA1, a right leg driving electrode RL, a first auxiliary right arm electrode RA1 and a main right arm electrode RA. The auxiliary left arm electrode and the auxiliary right arm electrode are referred to as regional electrodes, the number of the regional electrodes may be changed according to actual conditions, which is not limited to that the left and the right are respectively provided with two electrode, or the left and the right are respectively provided with one electrode, or the left and the right are correspondingly provided with a plurality of electrodes.

The present application also provides an embodiment of a fetal monitor including the detection probe in the above-mentioned embodiment.

An embodiment of the present application also provides a heart rate acquiring method which is applied to a detection probe including a main left arm electrode LA and a main right arm electrode RA as well as auxiliary left arm electrodes and auxiliary right arm electrodes. In the heart rate acquiring method, the specific numbers of the auxiliary left arm electrodes and the auxiliary right arm electrodes are not specifically limited, and the numbers of the auxiliary left arm electrodes and the auxiliary right arm electrodes may be changed according to actual conditions.

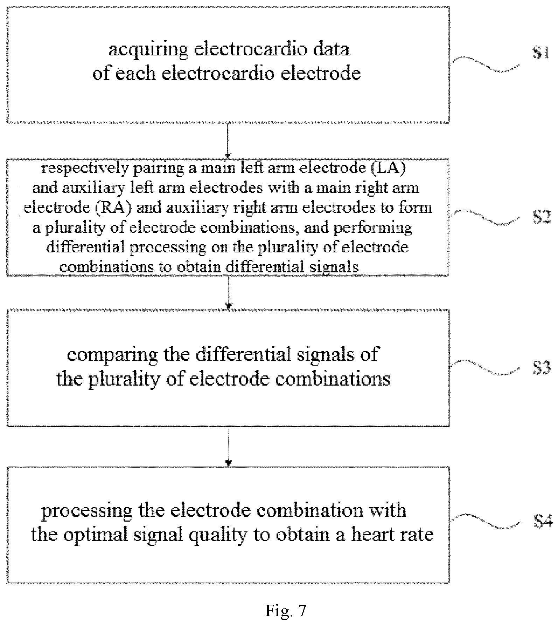

As shown in Fig. 7, the heart rate acquiring method mainly includes the steps.

Step S1: electrocardio data of each electrocardio electrode is acquired.

The acquisition of the electrocardio data of each electrocardio electrode may be achieved by using an acquisition circuit in the prior art or a working circuit as shown in Fig. 4.

Step S2: a main left arm electrode LA and auxiliary left arm electrodes are respectively paired with a main right arm electrode RA and auxiliary right arm electrodes to form a plurality of electrode combinations, and differential processing is performed on the plurality of electrode combinations to obtain differential signals.

The auxiliary left arm electrodes differ from the main right arm electrode RA, so that signals different from differential signals of the main left arm electrode LA and the main right arm electrode RA may be acquired, and signals forming different angles with an electrocardio axis may be acquired.

Similarly, the main left arm electrode LA differs from the auxiliary right arm electrodes, so that signals different from differential signals of the main left arm electrode LA and the main right arm electrode RA may be acquired, and signals forming different angles with the electrocardio axis may be acquired.

Therefore, a plurality of electrode combinations of which lead axes and electrocardio axes form different included angles may be formed by respectively pairing the main left arm electrode LA and the auxiliary left arm electrodes with the main right arm electrode RA and the auxiliary right arm electrodes, thereby covering different electrocardio axis ranges to acquire more electrocardio signals which may be selected.

Step S3: the differential signals of the plurality of electrode combinations are compared.

Generally, if an included angle of the lead axis and the electrocardio axis in a three-dimensional space is smaller, the electrocardio signals are forward and relatively large. Therefore, signal quality such as the strength and/or amplitude of each differential signal is compared. The included angle of the lead axis and the electrocardio axis of the electrode combination in the three-dimensional space may be judged according to the signal quality.

Step S4: the electrode combination with the optimal signal quality is processed to obtain a heart rate.

After comparison in the step S3, the electrode combination with the optimal signal quality may be achieved. The included angle of the lead axis and the electrocardio axis of the electrode combination in the three-dimensional space is minimum. The waveform size of an ECG picked up by the electrode is decided by the length of a projection of a mean QRS vector (QRS axis) on the lead axis, and therefore, a heart rate result acquired by adopting the electrode combination with the optimal signal quality is optimal.

By design, signals forming different angles with the electrocardio axis may be acquired by pairing and comparison with specific to the position of the electrocardio electrode, and an optimal electrocardio conducting direction is automatically adjusted, so that it is ensured that optimal signals are acquired, the stability and reliability of the overall monitoring process are guaranteed, and influences brought by artificial operation are reduced.

Optionally, in some embodiments of the present application, in order to improve the common-mode rejection (CMR) performance of a system, the electrocardio electrode further includes a right leg driving electrode RL.

The electrocardio electrode may also introduce an environmental electric signal such as an alternating current power supply, a safety system and radio-frequency interference (RFI) while acquiring an electrocardio signal on the surface of a human body, so that the electrocardio signal is amplified and displayed. However, a common-mode voltage does not provide any useful information about a heart, and in fact, the measuring precision may be affected. In a heart rate acquisition process, it has to be ensured that common-mode interference is rejected while a target signal, that is, a differential-mode ECG voltage is responded. The ability of rejecting a large common-mode signal in the case that there is a small differential signal is the CMR performance of the system.

The working principle of the right leg driving electrode is that a common-mode voltage on the surface of a human body is acquired by the right leg driving electrode RL and is then returned to the human body in a negative feedback amplification manner, so that the effect of neutralizing common-mode interference is achieved, and the common-mode voltage is rejected fundamentally.

Therefore, in order to improve the CMR ability of the system, the heart rate acquiring method in the present embodiment further includes:
a common-mode voltage is acquired by the right leg driving electrode RL; and
a rejection voltage matched with the common-mode voltage is transmitted to the main left arm electrode LA and the main right arm electrode RA

In some implementation solutions, the right leg driving electrode RL may be omitted.

For the detection probe including the seven electrocardio electrodes, an implementation of the seven corresponding electrocardio electrodes in the step S2 includes:
differential processing is performed on the main left arm electrode LA and the main right arm electrode RA to obtain differential signals;
differential processing is performed on the main left arm electrode LA and the first auxiliary right arm electrode RA1 to obtain differential signals;
differential processing is performed on the main left arm electrode LA and the second auxiliary right arm electrode RA2 to obtain differential signals;
differential processing is performed on the first auxiliary left arm electrode LA1 and the main right arm electrode RA to obtain differential signals; and
differential processing is performed on the second auxiliary left arm electrode LA2 and the main right arm electrode RA to obtain differential signals.

By such a design, the first auxiliary left arm electrode LA1 differs from the main right arm electrode RA, and the second auxiliary left arm electrode LA2 differs from the main right arm electrode RA, so that signals different from differential signals of the main left arm electrode LA and the main right arm electrode RA may be acquired, and signals forming different angles with the electrocardio axis may be acquired.

The main left arm electrode LA differs from the first auxiliary right arm electrode RA1, and the main left arm electrode LA differs from the second auxiliary right arm electrode RA2, so that signals different from differential signals of the main left arm electrode LA and the main right arm electrode RA may be acquired, and signals forming different angles with the electrocardio axis may be acquired.

A lead axis formed from the main left arm electrode LA and the second auxiliary right arm electrode RA2 is perpendicular to a lead axis formed from the main right arm electrode RA and the second auxiliary left arm electrode LA2, so that detection angles of a combination of the main left arm electrode LA and the second auxiliary right arm electrode RA2 and a combination of the second auxiliary left arm electrode LA2 and the main right arm electrode RA are 90 DEG, and then, the standard range of the electrocardio axis may be covered.

In the present solution, the number of the regional electrodes may be set as that the left and the right are respectively provided with one or more regional electrodes. The corresponding heart rate acquiring method may be adaptively changed according to the above-mentioned solution, and a pairing processing method is the same as that in the above-mentioned solution so as not to be described repeatedly.

Optionally, in some embodiments of the present application, the step that the electrocardio signal of each electrocardio electrode is acquired further includes:
electrocardio data is subjected to filtering preprocessing, wherein the filtering preprocessing includes: baseline shift, power frequency interference and myoelectric interference are filtered and removed; and QRS wave components are protruded. The high-pass filtering cut-off frequency is appropriately increased, and the higher-frequency baseline shift relatively easily introduced by the apparatus is rejected.

A R wave detection threshold baseline is adjusted according to signal amplitude information so that small-amplitude R waves may be successfully detected.

A heartbeat position is detected to acquire an RR interval, and it is determined whether the electrocardio data is effective according to the RR interval.

Optionally, in some embodiments of the present application, the step that the electrode combination with the optimal signal quality is processed to obtain the heart rate includes:
movement artifact interference which is possibly introduced is recognized according to signal peak information; and
at an interference section, a more accurate R wave position is selected according to a current heart rate baseline by using a peak spacing detection method. False detection easily caused by wave peak measurement on the interference section is corrected with reference to an interference recognition result.

An embodiment of the present application further provides a heart rate acquiring apparatus including:
an acquisition module, configured to acquire electrocardio data of each electrocardio electrode;
a differential module, configured to respectively pair a main left arm electrode and auxiliary left arm electrodes with a main right arm electrode and auxiliary right arm electrodes to form a plurality of electrode combinations and perform differential processing on the plurality of electrode combinations to obtain differential signals;
a comparison module, configured to compare the differential signals of the plurality of electrode combinations; and
a processing module, configured to process the electrode combination with the optimal signal quality to obtain a heart rate.

Obviously, the above-mentioned embodiments are merely examples for clear description, rather than limitations on implementations. Those of ordinary skill in the art may also make other changes or alterations in other different modes on the basis of the above-mentioned description. It is unnecessary and unable to exhaustively list all the implementations, and apparent changes or alterations derived therefrom still fall within the protection scope of the present application.

## Claims

1. A detection probe, comprising
a probe body (2), and
a mounting structure (1), detachably mounted on the probe body (2) and having a heart rate sensor for heart rate detection, a socket (4) for data transmission, and a lead line (5) for connecting the heart rate sensor and the socket (4) arranged thereon.

2. The detection probe of claim 1, wherein the mounting structure (1) is a housing structure matched with a shell of the probe body (2) and is detachably buckled on the probe body (2).

3. The detection probe of claim 2, **characterized in that** the housing structure comprises
an acting surface (3) fitted with the probe body (2), which is fitted to a user during detection, and has the heart rate sensor arranged thereon; and
a connecting part connected with the acting surface (3), which wraps a peripheral edge of the probe body (2).

4. The detection probe of claim 3, **characterized in that** the heart rate sensor is an electrocardio electrode arranged on the acting surface (3).

5. The detection probe of claim 4, **characterized in that** the electrocardio electrode comprises a main left arm electrode (LA) and a main right arm electrode (RA) respectively configured to acquire electrocardio signals of a left arm and a right arm.

6. The detection probe of claim 5, **characterized in** further comprising at least one auxiliary left arm electrode configured to acquire an electrocardio signal in a left arm area and spaced for a set distance from the main left arm electrode (LA) and at least one auxiliary right arm electrode configured to acquire an electrocardio signal in a right arm area and spaced for a set distance from the main right arm electrode (RA).

7. The detection probe of claim 6, **characterized in that** two auxiliary left arm electrodes which are respectively a first auxiliary left arm electrode (LA1) and a second auxiliary left arm electrode (LA2) and two auxiliary right arm electrodes which are respectively a first auxiliary right arm electrode (RA1) and a second auxiliary right arm electrode (RA2) are respectively arranged.

8. The detection probe of claim 7, **characterized in that** a lead axis formed from the main left arm electrode (LA) and the second auxiliary right arm electrode (RA2) is perpendicular to a lead axis formed from the main right arm electrode (RA) and the second auxiliary left arm electrode (LA2).

9. The detection probe of any one of claims 4-8, **characterized in** further comprising a right leg driving electrode (RL) configured to improve the common-mode rejection ability of a system.

10. The detection probe of claim 3, **characterized in that** the lead line (5) is arranged between the acting surface (3) and the probe body (2), and the housing structure is provided with a through hole allowing the heart rate sensor to pass through.

11. The detection probe of claim 10, **characterized in that** the lead line (5) is a flexible printed circuit (FPC) line or is of a conductive structure which is printed on the side, facing the probe body (2), of the housing structure.

12. The detection probe of claim 1, **characterized in that** the heart rate sensor is a blood oxygen photoplethysmographic sensor.

13. The detection probe of claim 1, **characterized in that** the probe body (2) is an outer uterine contraction pressure probe, a fetal heart probe or an inner monitoring probe.

14. A fetal monitor, comprising the detection probe of any one of claims 1-13.
